# EUROPEAN PATENT SPECIFICATION

(11) **EP 1 558 227 B1**
(45) Date of publication and mention of the grant of the patent: **04.10.2006**
(21) Application number: 03807993.5
(22) Date of filing: 21.08.2003
(51) Int. Cl.: A61K 31/05, A61K 47/34, A61K 47/10, A61K 47/14, A61K 9/107, A61P 23/00

(54) **INJECTABLE 2,6-DIISOPROPYLPHENOL-CONTAINING ANESTHETIC COMPOSITION AND METHODS**
INJIZIERBARE 2,6-DIISOPROPYLPHENOL ENTHALTENDE ANÄSTHETISCHE ZUSAMMENSETZUNG UND VERFAHREN
COMPOSITION ANESTHESIQUE INJECTABLE CONTENANT DU 2,6-DIISOPROPYLPHENOL ET METHODES ASSOCIEES

(30) Priority: 08.10.2002 KR 20020061260
(43) Date of publication of application: 03.08.2005
(73) Proprietor: DAEWON PHARM. CO., LTD., Kwangjin-gu 143-838 Seoul (KR)
(72) Inventor: JEE, Ung-Kil, Seoul 137-907 (KR)
(74) Representative: Schweitzer, Klaus
(86) International application number: PCT/KR2003/001690
(87) International publication number: WO 2004/032910

(56) References cited:
- WO-A1-00/59475
- WO-A1-00/78301
- WO-A2-02/09671
- KR-A- 2001 055 736

## Description

### TECHNICAL FIELD

This invention relates to a pharmaceutical composition, which is parenterally injectable in humans or animals, for inducing or maintaining anesthesia i.e. an injectable anesthetic composition in an aqueous phase comprising 2,6-diisopropylphenol (i.e., propofol) as an active ingredient.

### BACKGROUND ART

2,6-Diisopropyl phenol, which is used as an anesthetic, has a lipophilic character and is thus able to easily penetrate the blood-brain barrier. Owing to its lipophilic character, 2,6-diisopropylphenol is water-insoluble, which has resulted in difficulty in developing a formulation for intravenous injection.

At present, an injectable formulation comprising propofol emulsified with soybean oil, phospholipid, and glycerin is commercially available from AstraZeneca. This injectable emulsion is problematic because its milky appearance makes it difficult to detect impurities with the naked eye. Further, the relatively large particle size (>100 µm) can lead to formation of thrombi in capillaries and peripheral blood vessels.

U.S. Patent Nos. 4,056,635 and 4,798,846 disclose pharmaceutical compositions for general anesthesia, which are parenterally administrable to humans or animals, comprising a surfactant, such as CREMOPHOR EL® or polysorbate 80 (TWEEN 80® ), and water-immiscible solvents, such as ethyl oleate or castor oil, and an additional solvent, such as ethanol, polyethylene glycol, or propylene glycol. These pharmaceutical compositions, however, have significant drawbacks in terms of causing adverse effects, and hypersensitive responses against CREMOPHORS® can be induced in animals or humans.

Use of polysorbate 80 as a surfactant is disclosed in International Publication No. WO97/10814, however, the problem of hypersensitivity is also found in anesthetics containing polysorbate 80.

WO 00 78301 discloses a microemulsion of propofol. The formulation does not include Cremophor EL® which is considered to be allergenic.

Thus, while prior art propofol-containing products and methods of use thereof are known and are generally suitable for their limited purposes, they possess certain inherent deficiencies that detract from their overall utility for anesthesia. In view of the foregoing, it will be appreciated that providing an injectable propofol -containing anesthetic composition that is optically clear and does not produce a hypersensitive reaction would be a significant advancement in the art.

### DISCLOSURE OF THE INVENTION

It is an advantage of the present invention to provide an injectable anesthetic composition that does not induce a hypersensitive reaction.

It is another advantage of the invention to provide an optically clear injectable anesthetic composition that facilitates the detection of impurities or foreign matter contained therein with the naked eye.

In an embodiment of the invention, an injectable anesthetic composition comprises a microemulsion comprising a mixture of 2,6-diisopropylphenol, polyethylene glycol 660 hydroxystearate, tetrahydrofurfuryl alcohol polyethyleneglycol ether, and an aqueous medium. The composition can further comprise a surfactant selected from the group consisting of bile salts, lecithin, and mixtures thereof. The surfactant is present in an amount of 0.1 to 0.5% by weight. In another embodiment of the invention, the aqueous medium comprises a tonicity adjustment agent in an amount sufficient to obtain an isotonic condition corresponding to blood plasma. Tonicity adjustment agents include trehalose, glucose, fructose, glycerol, sorbitol, mannitol, sucrose, xylitol, sodium chloride, and mixtures thereof. In certain embodiments of the invention, the composition comprises 1 to 2% by weight of 2,6-diisopropylphenol, 5 to 10% by weight of polyethylene glycol 660 hydroxystearate, 10 to 25% by weight of tetrahydrofurfuryl alcohol polyethyleneglycol ether, and 63-84% by weight of the aqueous medium.

Another embodiment of the invention relates to a method of making an injectable anesthetic composition comprising:
(a) mixing polyethylene glycol 660 hydroxystearate with the aqueous medium to result in an aqueous mixture and heating and then cooling the aqueous mixture to room temperature to result in an aqueous solution;
(b) adding 2,6-diisopropylphenol to tetrahydrofurfuryl alcohol polyethyleneglycol ether to result in an oil-phase mixture and heating and then cooling the oil-phase mixture to room temperature to result in an oil-phase solution;
(c) mixing the aqueous solution and the oil-phase solution with stirring to result in a stirred mixture; and
(d) heating the stirred mixture with additional stirring and then cooling to room temperature to result in a microemulsion, thereby resulting in the injectable anesthetic composition.

Still another embodiment of the invention relates to a composition for anesthetizing an animal or human comprising injecting the animal or human with an amount of an anesthetic composition effective for inducing or maintaining anesthesia, wherein the composition comprises a microemulsion comprising a mixture of 2,6-diisopropylphenol, polyethylene glycol 660 hydroxystearate, tetrahydrofurfuryl alcohol polyethyleneglycol ether, and an aqueous medium.

### DETAILED DESCRIPTION OF THE INVENTION

It must be noted that, as used in this specification and the appended claims, the singular forms "a," "an," and "the" include plural referents unless the context clearly dictates otherwise. Thus, for example, reference to an anesthetic composition comprising "a surfactant" includes a mixture of one or more of such surfactants, reference to "an aqueous medium" includes reference to two or more of such aqueous media, and reference to "the thickening agent" includes reference to a mixture of two or more of such thickening agents.

In describing and claiming the present invention, the following terminology will be used in accordance with the definitions set out below.

As used herein, "consisting of" and grammatical equivalents thereof exclude any element, step, or ingredient not specified in the claim.

As used herein, a "pharmaceutically acceptable" component is one that is suitable for use with humans and/or animals without undue adverse side effects (such as toxicity, irritation, and allergic response) commensurate with a reasonable benefit/risk ratio. Preferably, as used herein, the term "pharmaceutically acceptable" means approved by a regulatory agency of the Federal or a state government or listed in the U.S. Pharmacopeia or other generally recognized pharmacopeia for use in animals, and more particularly in humans.

As used herein, "aqueous medium" means a water-containing liquid, which may also contain salts, buffers, pH-adjusting agents, tonicity adjustment agents.

As used herein, "bile salts" are pharmaceutically acceptable salts of cholic acid, deoxycholic acid, chenodeoxycholic acid, lithocholic acid, ursocholic acid, ursodeoxycholic acid, isoursodeoxycholic acid, lagodeoxycholic acid, glycocholic acid, taurocholic acid, glycodeoxycholic acid, glycochenodeoxycholic acid, dehydrocholic acid, hyocholic acid, hyodeoxycholic acid, and the like, and mixtures thereof.

As used herein, "optically clear" and similar terms mean that the composition exhibits a transmittance at 660 nm of greater than 90%, typically greater than 94%, and more typically greater than 97%.

As used herein, "PBS" means phosphate buffered saline, i.e., 0.01 M Na₂HPO₄, 0.15 M NaCl, pH 7.2.

Since the anesthetic composition of the present invention is intended to be administered to a warm-blooded animal, including humans, the ingredients should be pharmaceutically acceptable for administration to animals and humans.

The present invention is directed to an injectable anesthetic composition comprising a microemulsion comprising 2,6-diisopropylphenol as an active ingredient and the hydrophilic surfactant, polyethylene glycol 660 hydroxystearate (CAS No. 70142-34-6), and the cosurfactant and cosolvent, tetrahydrofurfuryl alcohol polyethyleneglycol ether (CAS No. 31692-85-0), wherein the microemulsion is formed by mixing these ingredients with an aqueous medium.

2,6-Diisopropylphenol, which is a widely used injectable anesthetic, is typically added to pharmaceutical compositions in an amount of 1-2% by weight for use in general anesthesia. If 2,6-diisopropylphenol is added in an amount of less that 1% by weight, sufficient anesthesia may not be achieved, in animal or human subjects. If the added amount of 2,6-diisopropylphenol exceeds 2% by weight, then adverse effects may occur owing to overdosing of the anesthetic agent. '

Used in the present invention as a surfactant, polyethylene glycol 660 hydroxystearate is commercially available as SOLUTOL® (BASF). For example, the product known as SOLUTOL HS 15® is known and commercially available.

In an embodiment of the invention, the surfactant polyethylene glycol 660 hydroxystearate is contained in the injectable anesthetic agent in an amount of 5-10% by weight, but its content is not limited to this range. That is, it is possible for the surfactant to be added in an amount of less than 5% or more than 10%. However, taking into consideration the range of content of the active ingredient, 2,6-diisopropylphenol, the content of the surfactant is typically greater than 5%. In addition, in the presence of 2,6-diisopropylphenol added in a maximum amount of 2% by weight, the surfactant displays good solubility even when added in an amount of 10% by weight.

Another additive, tetrahydrofurfuryl alcohol polyethyleneglycol ether is also commercially available as GLYCOFUROL® (GF), which is exemplified as GLYCOFUROL 75® .

In a typical embodiment of the present invention, the cosurfactant and cosolvent, tetrahydrofurfuryl alcohol polyethylene glycol ether, which acts as an auxiliary agent for dissolution of the active ingredient, 1,6-diisopropylphenol, is contained in the injectable anesthetic in an amount of 10-25% by weight, but its content is not limited to this range. When the amounts of the active ingredient, 2,6-diisopropylphenol, and the major surfactant, polyethylene glycol 660 hydroxystearate, are within the ranges as described above, an amount of the auxiliary agent, tetrahydrofurfuryl alcohol polyethyleneglycol ether within the described range is sufficient to obtain a clear injectable aqueous solution.

If desired, the injectable anesthetic agent according to the present invention can further include other surfactants, including a bile salt, such as sodium deoxycholate, and lecithin, and such selection of surfactants can be easily determined by one of ordinary skill in the art. Illustrative amounts of the bile salt and lecithin are in the range of 0.1-0.5% by weight.

Dispersion medium, which is an aqueous medium, may be pharmaceutically acceptable distilled water for parenteral injection or an aqueous solution prepared by adding a suitable amount of a tonicity adjustment agent to distilled water to give an isotonic condition. To maintain the isotonic condition, osmolality is 200-900 mOsmol/kg, and typically, 260-390 mOsmol/kg. Examples of the tonicity adjustment agent may include trehalose, glucose, fructose, glycerol, sorbitol, mannitol, sucrose, xylitol, sodium chloride, and mixtures thereof.

Besides the above ingredients, additives commonly used in the art may be used in the present invention. For example, the injectable anesthetic agent may include a liquid excipient, which is exemplified as ethanol, propylene glycol, glycerol, triethylene glycol, polyethylene glycol, and mixtures thereof. Also, a pH regulator may be used to adjust the pH in the range of 5.5 to 9.5, and examples of pH regulators include citric acid, acetate, phosphoric acid, ascorbic acid, gluconic acid, succinic acid, tartaric acid, lactic acid, and salts thereof, and mixtures thereof.

In addition, the injectable anesthetic agent may further include any of the following additives in a pharmaceutically acceptable amount: a thickening agent, an absorbent, a light stabilizer, a crystallization inhibitor, a complexing agent, an antioxidant, and an antiseptic. Illustrative thickening agents include methylcellulose, hydroxyethyl cellulose, sodium carboxymethyl cellulose, hydroxypropyl cellulose, polyvinylpyrrolidone, and mixtures thereof. Illustrative complexing agents include EDTA and salts thereof, phosphate, nitrate, acetate, citrate, and mixtures thereof. Illustrative antioxidants include ascorbic acid, sulfate compounds, L-cysteine, thiodipropionic acid, thiolactic acid, monothioglycerol, propyl galate, and mixtures thereof. Illustrative antiseptics include methyl *p*-oxybenzoate, propyl *p*-oxybenzoate, PHB ester, chlorobutanol, benzyl alcohol, butanol, butane-1,3-diol, chlorohexidin salts, benzoic acid and its salts, sorbic acid, and mixtures thereof.

The injectable anesthetic agent of a microemulsion type comprising the above ingredients according to the present invention illustratively and typically has a particle size of 15-35 nm.

In accordance with the present invention, there is provided a method of preparing such an injectable anesthetic composition containing 2,6-diisopropylphenol (generic name: propofol) as an active ingredient, which is homogeneously dispersed in an aqueous medium to give a clear emulsion, comprising (1) dissolving Solutol® in an aqueous medium by adding it to distilled water for parenteral injection or an aqueous solution containing a tonicity adjustment agent and heating, for example to 40-80°C or more typically 50-70°C, and then cooling the resulting mixture to room temperature to give an aqueous solution; (2) adding an effective amount of propofol to GLYCOFUROL® commonly used in an injectable preparation, heating to 40-80°C or more typically 50-70°C with stirring, and then cooling the resulting mixture to room temperature to give an oil-phase solution; (3) adding a suitable amount of the oil-phase solution into the aqueous solution at room temperature and then mixing the combination with stirring to allow reactions between the compounds; and (4) heating the reaction mixture again, illustratively at 40-80°C and more typically at 50-70°C with stirring, and then cooling to room temperature. Step 4 is usually repeated three or more times to produce a clear microemulsion.

Since a microemulsion is easily formed just by mixing with stirring according to the method of the present invention, the injectable anesthetic composition of a clear emulsion type according to the present invention can be simply prepared without use of high-cost equipment, such as a homogenizer or a microfluidizer, which are commonly used in the art.

The present invention will be explained in more detail with reference to the following examples in conjunction with the accompanying drawings.

As described above, the presently described injectable anesthetic composition does not induce hypersensitivity in animals or humans, and is optically clear, allowing detection of impurities with the naked eye, thus making it possible to prevent adverse effects from such impurities.

### Example 1

First, 7.5 g of Solutol HS 15® (BASF) was added to 50 ml distilled water for parenteral injection, and the mixture was heated at 60°C to dissolve Solutol HS® in the aqueous medium, and the resulting mixture was then cooled to room temperature to give an aqueous solution. Separately, 15 g of Glycofurol 75® (commercially available from GF) was mixed with 1 g of propofol with heating, and the resulting mixture was then cooled to room temperature to give an oil-phase solution. The oil-phase solution was added to the water-phase solution little by little with stirring at room temperature. After the addition was ended, the mixture was well mixed with stirring at 50 to 80°C, and then cooled to room temperature. The agitation at 50-80°C and cooling steps were performed three additional times, resulting in formation of a clear microemulsion.

Thereafter, 26.5 ml of 1/15 M phosphate buffered saline (pH 7.4) was added to the microemulsion, thus giving 100 ml of a 1% injectable preparation, which contains propofol in an amount of 1% by weight.

Using conventional methods known in the art, the resulting injectable preparation was analyzed for optical clarity, pH, particle size, and zeta-potential, and the results are give in Table 1.

### Example 2

To investigate the effects of the drug propofol on the physical and chemical properties of injectable preparations when the amount of propofol is increased, another injectable preparation was prepared according to the same procedure as in Example 1, except for addition of 1.1 g propofol, and production of an injectable preparation of total volume of 110 ml, by addition of 10 ml more distilled water.

Using conventional methods known in the art, the resulting injectable preparation was analyzed for optical clarity, pH, particle size, and zeta-potential, and the results are set out in Table 1.

### Example 3

An injectable preparation was prepared according to the same procedure as in Example 1, except for use of a 10% dextrose solution as the aqueous medium.

Using conventional methods known in the art, the resulting injectable preparation was analyzed for optical clarity, pH, particle size, and zeta-potential, and the results are give in Table 1.

### Example 4

An injectable preparation was prepared according to the same procedure as in Example 2, except for use of a 10% dextrose solution as an aqueous medium.

Using conventional methods known in the art, the resulting injectable preparation was analyzed for optical clarity, pH, particle size, and zeta-potential, and the results are give in Table 1.

| Table 1. Transmittance, pH, particle size, and zeta-potentials of injectable preparations in Examples 1 to 4. | | | | | |
|---|---|---|---|---|---|
| Preparation | Transmittance (%) | | pH | Particle size (nm) Zeta-potential (mV) | |
| | 540 nm | 660 nm | | | |
| Example 1 | 96.1 | 97.75 | 7.71 | 16.7 | -2.41--2.56 |
| Example 2 | 95.35 | 97.12 | 7.71 | 17.5 | -3.85--4.18 |
| Example 3 | 97.05 | 98.51 | 7.60 | 16.5 | -4.02--4.05 |
| Example 4 | 96.86 | 98.53 | 7.63 | 16.9 | -2.27--2.88 |

### Examples 5-8

First, 7.5 g of Solutol HS® 1 (BASF) was added to 50 ml of a 10% dextrose solution, and the mixture was heated to 50 to 80°C to dissolve the Solutol HS® 15 in the aqueous medium. The resulting mixture was then cooled to room temperature to give an aqueous solution. Separately, 15 g of Glycofurol 75® (GF) was mixed with 250 mg of a bile salt (sodium deoxycholate) and 500 mg of 99% egg lecithin, and the mixture was heated at 50-80°C to dissolve the ingredients. Next, 1 g (Example 5), 1.1 g(Example 6), 1.2 g (Example 7), or 1.3 g (Example 8) of propofol was added to the resulting mixture and dissolved completely, thus giving an oil-phase solution. The oil-phase solution was added to the aqueous solution little by little with stirring at room temperature, and the resulting mixture was heated at 60°C for 5 minutes with agitation and then cooled to room temperature. The agitation at 60°C and cooling steps were repeated three more times, resulting information of a clear microemulsion.

Thereafter, 25 ml of 1/15 M phosphate buffered saline (pH 7.4) was added to the microemulsion, thus giving a 1% injectable preparation, to which an aqueous medium was added according to intended use.

Using conventional methods known in the art, the resulting injectable preparation was analyzed for optical clarity and particle size, and the results are give in Table 2.

### Examples 9-12

Injectable preparations of Examples 9 to 12 were prepared according to the same procedure as in Examples 5-8, except for use of a 20% trehalose solution as an aqueous medium.

Using conventional methods known in the art, the resulting injectable preparation was analyzed for optical clarity and particle size, and the results are given in Table 2.

### Examples 13-16

Injectable preparations of Examples 13 to 16 were prepared according to the same procedure as Examples 5-8, except for use of 10 ml of a 10% trehalose solution as aqueous medium.

Using conventional methods known in the art, the resulting injectable preparation was analyzed for optical clarity and particle size, and the results are given in Table 2.

| Table 2. Optical clarity and particle sizes of injectable preparations of Examples 5-16 | | |
|---|---|---|
| Injectable Preparation | Optical clarity (%, at 660 nm) | Particle size (nm) |
| Example 5 | 99.16 | 26.2 |
| Example 6 | 98.95 | 25.5 |
| Example 7 | 99.38 | 24.6 |
| Example 8 | 98.75 | 26.0 |
| Example 9 | 100.00 | 33.6 |
| Example 10 | 99.83 | 34.3 |
| Example 11 | 99.74 | 29.7 |
| Example 12 | 99.03 | 34.2 |
| Example 13 | 98.71 | 29.1 |
| Example 14 | 99.57 | 27.6 |
| Example 15 | 98.92 | 25.2 |
| Example 16 | 99.14 | 25.6 |

### Example 17-19

According to Table 3, phosphate buffered saline (pH 7.4) was used as an aqueous medium in Example 17, a 10% trehalose solution was used in Example 18, and distilled water for injection was used in Example 19. An oil-phase solution in each of Experiments 17-19 was prepared according to the procedure of Example 7. A 1% injectable preparation in each of Examples 17-19 was prepared according to the procedure of Example 1 except that a final volume of 120 ml was obtained.

| Table 3. Compositions of injectable preparations of Examples 17 to 19 | | | |
|---|---|---|---|
| | Example 17 | Example 18 | Example 19 |
| Aqueous solutions | 50 ml PBS | 50 ml 10% trehalose | 50 ml distilled water |
| | 7.5 g Solutol ® | 7.5 g Solutol® | 7.5 g Solutol ® |
| Oil-phase solutions | 15 g Glycofurol® | 15 g Glycofurol® | 15 g Glycofurol® |
| | 250 mg SDC | 250 mg SDC | 250 mg SDC |
| | 500 mg 99% egg lecithin | 500 mg 99% egg lecithin | 500 mg 99% egg lecithin |
| | 1.2 g propofol | 1.2 g propofol | 1.2 g propofol |
| PBS | q.s. | q.s. | q.s. |
| Total Volume | 120 ml | 120 ml | 120 ml |

Using conventional methods known in the art, the resulting injectable preparations were analyzed for pH, optical clarity, viscosity, and particle size, and the results are given in Table 4.

| Table 4 | | | | |
|---|---|---|---|---|
| Injectable Preparation | pH | Optical Clarity at 660 nm (%) | Viscosity (mPa·s) | Particle size (nm) |
| Example 17 | 7.6 | 98.7 | 0.8747 | 26.5 |
| Example 18 | 7.4 | 97.9 | 0.8764 | 27.1 |
| Example 19 | 7.5 | 99.0 | 0.8705 | 23.2 |

### Example 20

According to Table 5, a dextrose solution was used as an aqueous medium instead of trehalose solution, and an oil-phase solution was prepared by reducing an amount of egg lecithin to 250 mg and adding 1.1 g of the drug propofol. In each Example, a 1% injectable preparation was formulated according to procedure of Example 1 except that a final volume of 110 ml was obtained.

| Table 5 Compositions of injectable preparations | | | |
|---|---|---|---|
| | Example 17 | Example 18 | Example 19 |
| Aqueous solutions | 50 ml distilled water | 50ml 10% dextrose | 50 ml 10% dextrose |
| | 7.5 g Solutol® | 7.5 g Solutol® | 7.5 g Solutol® |
| Oil-phase solutions | 15 g Glycofurol® | 15 g Glycofurol® | 15 g Glycofurol® |
| | 250 mg SDC | 250 mg SDC | 250 mg SDC |
| | 250 mg 99% egg lecithin | 250 mg 99% egg lecithin | 250 mg 99% egg lecithin |
| | 1.1 g propofol | 1.1 g propofol | 1.1 g propofol |
| PBS | q.s. | q.s. | q.s. |
| Total Volume | 110 ml | 110 ml | 110 ml |

Using conventional methods known in the art, the resulting injectable preparations were analyzed for pH and optical clarity, and the results are give in Table 6.

| Table 6 | | |
|---|---|---|
| Injectable Preparation | pH | Optical Clarity at 660 nm (%) |
| Example 17 | 7.5 | 96.04 |
| Example 18 | 6.7 | 94.93 |
| Example 19 | 6.9 | 97.24 |

### Experimental Example 1

Test for an anesthetic effect of the injectable preparation. The injectable preparation of Example 5 was compared with the oil-based emulsion DIPRIVAN in terms of anesthetic effect according to their administered amounts, as well as on blood pressure and respiration in rabbits. Three rabbits of about 3 kg of body weight were used in each case. After immobilizing rabbits on a fixed board, a 24-ga. venous catheter was inserted into the ear vein, and an anesthetic agent was injected into the ear vein through the catheter. After recording baseline levels of the venous pressure at the rabbit ear, the anesthetic agent was injected at a rate of 1 ml/kg/hr. After 10 minutes, the venous pressure at the rabbit ear was again recorded. Thereafter, the injected amount of the anesthetic agent was increased to 2 ml/kg/hr, 4 ml/kg/hr, 6 ml/kg/hr, 8 ml/kg/hr, and 10 ml/kg/hr at intervals of 10 minutes.

The anesthetic effect of the treatment was evaluated by inserting a 24-gauge arterial catheter into the ear artery, and the arterial pressure at the rabbit ear was measured. Induction of anesthesia in rabbits was evaluated by stimulating the cornea with gauze, or investigating response of the end of the rabbit's nose upon being pricked with a needle, which is the so-called pinprick stimulation method.

The results are give in Tables 7 and 8.

| Table 7. Effect of the injectable preparation of Example 5 on induction of anesthesia in rabbits. | | | | | | | | |
|---|---|---|---|---|---|---|---|---|
| | Rabbit No. | Baseline | Dose rate (ml/kg/hr) | | | | | |
| | | | 1 | 2 | 4 | 6 | 8 | 10 |
| BP (S/D) | 1 | 105/70 | 105/71 | 95/70 | 79/61 | 73/61 | 61/47 | 68/48 |
| | 2 | 100/65 | 94/61 | 89/60 | 80/70 | 74/65 | 71 /62 | 69/58 |
| | 3 | 100/70 | 92/64 | 90/65 | 87/65 | 107/83 | 87/62 | 83/53 |
| Stimulation of the cornea | 1 | +++ | +++ | +++ | + | + | + | + |
| | 2 | +++ | +++ | +++ | ++ | + | + | + |
| | 3 | +++ | +++ | +++ | ++ | ++ | + | + |
| Pinprick stimulation | 1 | +++ | +++ | +++ | + | + | + | + |
| | 2 | +++ | +++ | +++ | ++ | + | + | + |
| | 3 | +++ | +++ | +++ | ++ | + | ∀ | - |
| Difficulty in breathing | 1 | - | - | - | - | - | - | + |
| | 2 | - | - | - | - | - | - | - |
| | 3 | - | - | - | - | - | - | - |

| Table 8. Effect of DIPRIVAN on induction of anesthesia in rabbits | | | | | | | | |
|---|---|---|---|---|---|---|---|---|
| | Rabbit No. | Baseline | Dose rate (ml/kg/hr) | | | | | |
| | | | 1 | 2 | 4 | 6 | 8 | 10 |
| BP (S/D) | 4 | 79/61 | 82/65 | 83/64 | 65/52 | 84/63 | 84/60 | 81/61 |
| | 5 | 100/66 | 110/69 | 92/70 | 85/73 | 82/66 | anesthetized | death |
| | 6 | 91/63 | 85/63 | 83/60 | 60/54 | 63/57 | 70/59 | 70/59 |
| Stimulation of the cornea | 4 | +++ | +++ | +++ | +++ | + | + | ∀ |
| | 5 | +++ | +++ | +++ | +++ | +++ | - | - |
| | 6 | +++ | +++ | ++ | + | + | + | ∀ |
| Pinprick stimula- | 4 | +++ | +++ | +++ | ++ | + | + | ∀ |
| | 5 | +++ | +++ | +++ | +++ | +++ | - | - |
| | 6 | +++ | +++ | ++ | + | + | + | ∀ |
| Difficulty in breathing | 4 | - | - | - | - | - | - | - |
| | 5 | - | - | - | - | - | - | death |
| | 6 | - | - | - | - | - | - | - |

As is apparent from the data in Tables 7 and 8, the injectable preparation of Example 5 has an anesthetic effect similar to that of DIPRIVAN.

### Experimental Example 2

Assay for stability of the injectable formulations of Examples 5 to 16. After being kept in cold storage for 90 days, the injectable preparations of Examples 5 to 16 were analyzed for stability using HPLC. The pump was a Waters model 510, and the detector was a Waters model 486. The column was an Intersil ODS 3.5 µm, 4.6 x 250 mm from GL Science. The mobile phase was acetonitrile:water:acetic acid (pH 2.0) in a ratio of 70:30:0.1. The sample size injected was 50 µℓ, and fractionation was carried out at a flow rate of 1.2 ml/min. Detection was at 276 nm.

The results of these HPLC assays showed that all of the formulations had stabilities in the range of 98 to 100%.

## Claims

1. An injectable anesthetic composition comprising a microemulsion comprising a mixture of 2,6-diisopropylphenol, polyethylene glycol 660 hydroxystearate, tetrahydrofurfuryl alcohol polyethyleneglycol ether, and an aqueous medium.

2. The composition of claim 1 further comprising a surfactant selected from the group consisting of bile salts, lecithin, and mixtures thereof.

3. The composition of claim 2, wherein the surfactant is a bile salt.

4. The composition of claim 2, wherein the surfactant is lecithin.

5. The composition of claim 2, wherein the surfactant is a mixture of a bile salt and lecithin.

6. The composition of one of claims 2 to 5, wherein the composition comprises 0.1 to 0.5% by weight of the surfactant.

7. The composition of claim 2, 3 or 5, wherein the bile salt is selected from the group consisting of pharmaceutically acceptable salts of cholic acid, deoxycholic acid, chenodeoxycholic acid, lithocholic acid, ursocholic acid, ursodeoxycholic acid, isoursodeoxycholic acid, lagodeoxycholic acid, glycocholic acid, taurocholic acid, glycodeoxycholic acid, glycochenodeoxycholic acid, dehydrocholic acid, hyocholic acid, hyodeoxycholic acid, and mixtures thereof.

8. The composition of one of claims 1 to 7, wherein the aqueous medium comprises a tonicity adjustment agent in an amount sufficient to obtain an isotonic condition corresponding to blood plasma.

9. The composition of claim 8, wherein the tonicity adjustment agent is a member selected from the group consisting of trehalose, glucose, fructose, glycerol, sorbitol, mannitol, sucrose, xylitol, sodium chloride, and mixtures thereof.

10. The composition of one of claims 1 to 9, wherein the composition comprises 1 to 2% by weight of 2, 6-diisopropylphenol.

11. The composition of one of claims 1 to 10, wherein the composition comprises 5 to 10% by weight of polyethylene glycol 660 hydroxystearate.

12. The composition of one of claims 1 to 11, wherein the composition comprises 10 to 25% by weight of tetrahydrofurfuryl alcohol polyethyleneglycol ether.

13. The composition of one of claims 1 to 12, further comprising a member selected from the group consisting of liquid excipients, pH regulators, thickening agents, absorbents, light stabilizers, crystallization inhibitors, complexing agents, antioxidants, antiseptics, and mixtures thereof.

14. The composition of claim 13 wherein the composition comprises a liquid excipient selected from the group consisting of ethanol, propylene glycol, glycerol, triethylene glycol, polyethylene glycol, and mixtures thereof.

15. The composition of claim 13 wherein the composition comprises a pH regulator selected from the group consisting of citric acid, acetate, phosphoric acid, ascorbic acid, gluconic acid, succinic acid, tartaric acid, lactic acid, and salts thereof, and mixtures thereof.

16. The composition of claim 13 wherein the composition comprises a thickening agent selected from the group consisting of methylcellulose, hydroxyethyl cellulose, sodium carboxymethyl cellulose, hydroxypropyl cellulose, polyvinylpyrrolidone, and mixtures thereof.

17. The composition of claim 13 wherein the composition comprises a complexing agent selected from the group consisting of EDTA and salts thereof, phosphate, nitrate, acetate, citrate, and mixtures thereof.

18. The composition of claim 13 wherein the composition comprises an antioxidant selected from the group consisting of ascorbic acid, sulfate compounds, L-cysteine, thiodipropionic acid, thiolactic acid, monothioglycerol, propyl galate, and mixtures thereof.

19. The composition of claim 13 wherein the composition comprises an antiseptic selected from the group consisting of methyl p-oxybenzoate, propyl p-oxybenzoate, PHB ester, chlorobutanol, benzyl alcohol, butanol, butane-1,3-diol, chlorohexidin salts, benzoic acid and its salts, sorbic acid, and mixtures thereof.

20. The composition of one of claims 1 to 19, wherein the composition exhibits a transmittance at 660 nm of greater than 90%.

21. A method of making an injectable anesthetic composition comprising: (a) mixing polyethylene glycol 660 hydroxystearate with an aqueous medium to result in an aqueous mixture and heating and then cooling the aqueous mixture to room temperature to result in an aqueous solution; (b) adding 2,6-diisopropylphenol to tetrahydrofurfuryl alcohol polyethyleneglycol ether to result in an oil-phase mixture and heating and then cooling the oil-phase mixture to room temperature to result in an oil-phase solution; (c) mixing the aqueous solution and the oil-phase solution with stirring to result in a stirred mixture; and (d) heating the stirred mixture with additional stirring and then cooling to room temperature to result in a microemulsion, thereby resulting in the injectable anesthetic composition.

22. The method of claim 21 wherein heating of the aqueous mixture, the oil-phase mixture, and the stirred mixture is carried out at 40-80°C.

23. Use of a composition according to claim 1 for the manufacture of a medicament for inducing or maintaining anesthesia in an animal or human.

24. A medicament comprising a microemulsion comprising a mixture of 2,6-diisopropylphenol, polyethylene glycol 660 hydroxystearate, tetrahydrofurfuryl alcohol polyethyleneglycol ether, and an aqueous medium for use, as an anesthetic.

## Patentansprüche

1. Injektionsanästhesiemittel, umfassend eine Mikroemulsion, die eine Mischung von 2,6-Diisopropylphenol, Polyethylenglykol-660-hydroxystearat, Tetrahydrofurfurylalkoholpolyethylenglykolether und ein wäßriges Medium enthält.

2. Mittel nach Anspruch 1, ferner umfassend ein Tensid aus der Gruppe bestehend aus Gallensalzen, Lecithin und Mischungen davon.

3. Mittel nach Anspruch 2, **dadurch gekennzeichnet, daß** es sich bei dem Tensid um ein Gallensalz handelt.

4. Mittel nach Anspruch 2, **dadurch gekennzeichnet, daß** es sich bei dem Tensid um Lecithin handelt.

5. Mittel nach Anspruch 2, **dadurch gekennzeichnet, daß** es sich bei dem Tensid um eine Mischung aus einem Gallensalz und Lecithin handelt.

6. Mittel nach einem der Ansprüche 2 bis 5, **dadurch gekennzeichnet, daß** es 0,1 bis 0,5 Gew.-% des Tensids enthält.

7. Mittel nach Anspruch 2, 3 oder 5, **dadurch gekennzeichnet, daß** das Gallensalz aus der Gruppe bestehend aus pharmazeutisch unbedenklichen Salzen von Cholsäure, Desoxycholsäure, Chenodesoxycholsäure, Lithocholsäure, Ursocholsäure, Ursodesoxycholsäure, Isoursodesoxycholsäure, Lagodesoxycholsäure, Glykocholsäure, Taurocholsäure, Glykodesoxycholsäure, Glykochenodesoxycholsäure, Dehydrocholsäure, Hyocholsäure, Hyodesoxycholsäure und Mischungen davon ausgewählt ist.

8. Mittel nach einem der Ansprüche 1 bis 7, **dadurch gekennzeichnet, daß** das wäßrige Medium ein tonussteuerndes Mittel in einer zum Erhalt eines Blutplasma entsprechenden isotonischen Zustands ausreichenden Menge enthält.

9. Mittel nach Anspruch 8, **dadurch gekennzeichnet, daß** das tonussteuernde Mittel ein Mitglied der Gruppe bestehend aus Trehalose, Glucose, Fructose, Glycerin, Sorbit, Mannit, Saccharose, Xylit, Natriumchlorid und Mischungen davon ist.

10. Mittel nach einem der Ansprüche 1 bis 9, **dadurch gekennzeichnet, daß** es 1 bis 2 Gew.-% 2,6-Diisopropylphenol enthält.

11. Mittel nach einem der Ansprüche 1 bis 10, **dadurch gekennzeichnet, daß** es 5 bis 10 Gew.-% Polyethylenglykol-660-hydroxystearat enthält.

12. Mittel nach einem der Ansprüche 1 bis 11, **dadurch gekennzeichnet, daß** es 10 bis 25 Gew.-% Tetrahydrofurfurylalkoholpolyethylenglykolether enthält.

13. Mittel nach einem der Ansprüche 1 bis 12, ferner umfassend ein Mitglied aus der Gruppe bestehend aus flüssigen Trägerstoffen, pH-Reglern, Verdickungsmitteln, Absorptionsmitteln, Lichtschutzmitteln, Kristallisationsinhibitoren, Komplexbildnern, Antioxidantien, Antiseptika und Mischungen davon.

14. Mittel nach Anspruch 13, **dadurch gekennzeichnet, daß** es einen flüssigen Trägerstoff aus der Gruppe bestehend aus Ethanol, Propylenglykol, Glycerin, Triethylenglykol, Polyethylenglykol und Mischungen davon enthält.

15. Mittel nach Anspruch 13, **dadurch gekennzeichnet, daß** es einen pH-Regler aus der Gruppe bestehend aus Citronensäure, Acetat, Phosphorsäure, Ascorbinsäure, Gluconsäure, Bernsteinsäure, Weinsäure, Milchsäure und Salzen davon und Mischungen davon enthält.

16. Mittel nach Anspruch 13, **dadurch gekennzeichnet, daß** es ein Verdickungsmittel aus der Gruppe bestehend aus Methylcellulose, Hydroxyethylcellulose, Natriumcarboxymethylcellulose, Hydroxypropylcellulose, Polyvinylpyrrolidon und Mischungen davon enthält.

17. Mittel nach Anspruch 13, **dadurch gekennzeichnet, daß** es einen Komplexbildner aus der Gruppe bestehend aus EDTA und Salzen davon, Phosphat, Nitrat, Acetat, Citrat und Mischungen davon enthält.

18. Mittel nach Anspruch 13, **dadurch gekennzeichnet, daß** es ein Antioxidans aus der Gruppe bestehend aus Ascorbinsäure, Schwefelverbindungen, L-Cystein, Thiodipropionsäure, Thiomilchsäure, Monothioglycerin, Propylgallat und Mischungen davon enthält.

19. Mittel nach Anspruch 13, **dadurch gekennzeichnet, daß** es ein Antiseptikum aus der Gruppe bestehend aus Methyl-p-oxybenzoat, Propyl-p-oxybenzoat, PHB-Ester, Chlorbutanol, Benzylalkohol, Butanol, Butan-1,3-diol, Chlorhexidinsalzen, Benzoesäure und deren Salzen, Sorbinsäure und Mischungen davon enthält.

20. Mittel nach einem der Ansprüche 1 bis 19, **dadurch gekennzeichnet, daß** es eine Durchlässigkeit bei 660 nm von mehr als 90 % aufweist.

21. Verfahren zur Herstellung eines Injektionsanästhesiemittels, bei dem man: (a) Polyethylenglykol-660-hydroxystearat mit einem wäßrigen Medium vermischt, wobei man eine wäßrige Mischung erhält, und die wäßrige Mischung erhitzt und dann auf Raumtemperatur abkühlt, wobei man eine wäßrige Lösung erhält; (b) 2,6-Diisopropylphenol zu Tetrahydrofurfurylalkoholpolyethylenglykolether gibt, wobei man eine Ölphasenmischung erhält, und die Ölphasenmischung erhitzt und dann auf Raumtemperatur abkühlt, wobei man eine Ölphasenlösung erhält; (c) die wäßrige Lösung und die Ölphasenlösung unter Rühren vermischt, wobei man eine gerührte Mischung erhält; und (d) die gerührte Mischung unter zusätzlichem Rühren erhitzt und dann auf Raumtemperatur abkühlt, wobei man eine Mikroemulsion erhält, was das Injektionsanästhesiemittel ergibt.

22. Verfahren nach Anspruch 21, **dadurch gekennzeichnet, daß** man das Erhitzen der wäßrigen Mischung, der Ölphasenmischung und der gerührten Mischung bei 40-80°C durchführt.

23. Verwendung eines Mittels nach Anspruch 1 zur Herstellung eines Arzneimittels zur Induktion oder Aufrechterhaltung von Anästhesie bei einem Tier oder einem Menschen.

24. Arzneimittel, umfassend eine Mikroemulsion, die eine Mischung von 2,6-Diisopropylphenol, Polyethylenglykol-660-hydroxystearat, Tetrahydrofurfurylalkoholpolyethylenglykolether und ein wäßriges Medium enthält, zur Verwendung als Anästhetikum.

## Revendications

1. Composition anesthésique injectable comprenant une microémulsion comprenant un mélange de 2,6-düsopropylphénol, d'hydroxystéarate de polyéthylène glycol 660, d'éther de polyéthylèneglycol d'alcool tétrahydrofurfurylique, et d'un milieu aqueux.

2. Composition selon la revendication 1, comprenant en outre un tensioactif sélectionné dans le groupe constitué par des sels biliaires, la lécithine, et des mélanges de ceux-ci.

3. Composition selon la revendication 2, dans laquelle le tensioactif est un sel biliaire.

4. Composition selon la revendication 2, dans laquelle le tensioactif est la lécithine.

5. Composition selon la revendication 2, dans laquelle le tensioactif est un mélange d'un sel biliaire et de lécithine.

6. Composition selon l'une des revendications 2 à 5, où la composition comprend de 0,1 à 0,5 % en poids du tensioactif.

7. Composition selon la revendication 2, 3 ou 5, dans laquelle le sel biliaire est sélectionné dans le groupe constitué par des sels pharmaceutiquement acceptables de l'acide cholique, de l'acide désoxycholique, de l'acide chénodésoxycholique, de l'acide lithocholique, de l'acide ursocholique, de l'acide ursodésoxycholique, de l'acide isoursodésoxycholique, de l'acide lagodésoxycholique, de l'acide glycocholique, de l'acide taurocholique, de l'acide glycodésoxycholique, de l'acide glycochénodésoxycholique, de l'acide déshydrocholique, de l'acide hyocholique, de l'acide hyodésoxycholique, et des mélanges de ceux-ci.

8. Composition selon l'une des revendications 1 à 7, dans laquelle le milieu aqueux comprend un agent d'ajustement de la tonicité en une quantité suffisante pour obtenir un état isotonique correspondant au plasma sanguin.

9. Composition selon la revendication 8, dans laquelle l'agent d'ajustement de la tonicité est un membre sélectionné dans le groupe constitué par le tréhalose, le glucose, le fructose, le glycérol, le sorbitol, le mannitol, le saccharose, le xylitol, le chlorure de sodium et des mélanges de ceux-ci.

10. Composition selon l'une des revendications 1 à 9, où la composition comprend de 1 à 2 % en poids de 2,6-diisopropylphénol.

11. Composition selon l'une des revendications 1 à 10, où la composition comprend de 5 à 10 % en poids d'hydroxystéarate de polyéthylène glycol 660.

12. Composition selon l'une des revendications 1 à 11, où la composition comprend de 10 à 25 % en poids d'éther de polyéthylèneglycol d'alcool tétrahydrofurfurylique.

13. Composition selon l'une des revendications 1 à 12, comprenant en outre un membre sélectionné dans le groupe constitué par des excipients liquides, des régulateurs de pH, des agents épaississants, des absorbants, des stabilisateurs contre la détérioration par la lumière, des inhibiteurs de cristallisation, des agents complexants, des antioxydants, des antiseptiques, et des mélanges de ceux-ci.

14. Composition selon la revendication 13, où la composition comprend un excipient liquide sélectionné dans le groupe constitué par l'éthanol, le propylène glycol, le glycérol, le triéthylène glycol, le polyéthylène glycol et des mélanges de ceux-ci.

15. Composition selon la revendication 13, où la composition comprend un régulateur de pH sélectionné dans le groupe constitué par l'acide citrique, un acétate, l'acide phosphorique, l'acide ascorbique, l'acide gluconique, l'acide succinique, l'acide tartarique, l'acide lactique et des sels de ceux-ci et des mélanges de ceux-ci.

16. Composition selon la revendication 13, où la composition comprend un agent épaississant sélectionné dans le groupe constitué par la méthylcellulose, l'hydroxyéthylcellulose, la carboxyméthyl cellulose de sodium, l'hydroxypropyl cellulose, la polyvinylpyrrolidone, et des mélanges de ceux-ci.

17. Composition selon la revendication 13, où la composition comprend un agent complexant sélectionné dans le groupe constitué par l'EDTA et des sels de celui-ci, un phosphate, un nitrate, un acétate, un citrate, et des mélanges de ceux-ci.

18. Composition selon la revendication 13, où la composition comprend un agent antioxydant sélectionné dans le groupe constitué par l'acide ascorbique, des composés de sulfate, la L-cystéine, l'acide thiodipropionique, l'acide thiolactique, le monothioglycérol, le galate de propyle, et des mélanges de ceux-ci.

19. Composition selon la revendication 13, où la composition comprend un antiseptique sélectionné dans le groupe constitué par le p-oxybenzoate de méthyle, le p-oxybenzoate de propyle, un ester de PHB, le chlorobutanol, l'alcool benzylique, le butanol, le butane-1,3-diol, des sels de chlorohexidine, l'acide benzoïque et ses sels, l'acide sorbique, et des mélanges de ceux-ci.

20. Composition selon l'une des revendications 1 à 19, où la composition présente une transmission à 660 nm supérieure à 90 %.

21. Procédé de fabrication d'une composition anesthésique injectable comprenant les étapes consistant à : (a) mélanger l'hydroxystéarate de polyéthylène glycol 660 à un milieu aqueux pour produire un mélange aqueux et chauffer et puis faire revenir le mélange aqueux à température ambiante pour obtenir une solution aqueuse ; (b) ajouter le 2,6-diisopropylphénol à l'éther de polyéthylèneglycol d'alcool tétrahydrofurfurylique pour produire un mélange à phase huileuse et chauffer et puis faire refroidir le mélange à phase huileuse à température ambiante pour obtenir une solution à phase huileuse ; (c) mélanger la solution aqueuse et la solution à phase huileuse en agitant pour obtenir un mélange agité ; et (d) chauffer le mélange agité en poursuivant l'agitation et puis faire refroidir à température ambiante pour obtenir une microémulsion, obtenant ainsi la composition anesthésique injectable.

22. Procédé selon la revendication 21, dans lequel le chauffage du mélange aqueux, du mélange à phase huileuse, et du mélange agité est réalisé à 40-80°C.

23. Utilisation d'une composition selon la revendication 1 pour la fabrication d'un médicament pour induire ou maintenir une anesthésie chez un animal ou un être humain.

24. Médicament comprenant une microémulsion comprenant un mélange de 2,6-düsopropylphénol, d'hydroxystéarate de polyéthylène glycol 660, d'éther de polyéthylèneglycol d'alcool tétrahydrofurfurylique, et d'un milieu aqueux pour une utilisation en tant qu'anesthésique.
